# EUROPEAN PATENT APPLICATION

(11) **EP 4 520 335 A1**
(43) Date of publication of application: **12.03.2025**
(21) Application number: 23799596.4
(22) Date of filing: 18.04.2023
(51) Int. Cl.: A61K 31/555, A61P 7/02, A61P 29/00

(54) **DO3A-BASED ANTITHROMBOTIC AGENT OR HEMOLYTIC AGENT CONTAINING GADOLINIUM COMPLEX**

(30) Priority: 02.05.2022 KR 20220054399
(71) Applicant: Etnova Therapeutics Corp., Gyeonggi-do 13207 (KR)
(72) Inventor: CHANG, Sha Joung, Seongnam-si Gyeonggi-do 13458 (KR); KIM, Yeoun Hee, Daegu 41521 (KR); CHOI, Ga Ram, Daegu 41526 (KR); PARK, Hyun Jin, Daegu 41416 (KR); KIM, Ye Sl, Gapyeong-gun Gyeonggi-do 12447 (KR); CHOI, Min Jin, Daegu 41416 (KR); HWANG, Seong Hwan, Hwaseong-si, Gyeonggi-do 18392 (KR)
(74) Representative: Laine IP Oy
(86) International application number: PCT/KR2023/005214
(87) International publication number: WO 2023/214713

(57) **Abstract**

Disclosed is applicability in novel uses of a DO3A-based material containing a gadolinium complex as an antithrombotic agent or a coagulant, on the basis of a discovery finding that the DO3A-based material containing the gadolinium complex which has been used as an anti-inflammatory agent has a novel effect of suppressing a thrombus induced by thrombin or dissolving blood, without being involved in the function of platelet action which stops bleeding at a damaged or injured area.

## Description

### FIELD

The present disclosure relates to a DO3A-based pharmaceutical composition containing a gadolinium complex. More specifically, the present disclosure relates to the pharmaceutical composition conventionally known as an anti-inflammatory agent which is capable of providing a novel effect of thrombus inhibition or thrombolysis.

### DESCRIPTION OF RELATED ART

A non-steroidal anti-inflammatory drug (NSAID) inhibits COX-1 enzyme and COX-2 enzyme involved in prostaglandin synthesis, and thus, cause analgesic, anti-inflammatory, and antipyretic effects. COX-1 activates prostaglandin and thromboxane A2 to protect the gastric mucosa, maintain renal function, and platelet hemostasis. COX-2 activates inflammatory mediators and cytokines of various tissues such as vascular endothelial cells, thereby causing pain and inflammation.

NSAIDs are classified into non-selective NSAIDs and COX-2 selective NSAIDs based on selectivity of these COX substances. The non-selective NSAID inhibits the COX-1 as well and thus inhibits the protective effect of the gastric mucosa and causes antiplatelet effects, thereby frequently causing gastrointestinal side effects such as peptic ulcers. This is a fatal weakness of the non-selective NSAID. Thus, the COX-2 selective NSAID has emerged with the expectation that it could resolve various side effects caused by COX-1 blockade while maintaining anti-inflammatory and analgesic effects by selectively inhibiting only COX-2.

While the COX-2 selective NSAID is known to reduce the incidence of gastrointestinal complications, there is still controversy over cardiovascular and nephrotoxic side effects thereof. When the NSAID inhibits COX-2, prostacyclin production is inhibited, which reduces the antiplatelet effect, causes sodium and water retention, and further promotes platelet coagulation by COX-1, which increases the risk of thrombus and embolism, which increases the occurrence of cardiovascular events.

In this regard, diflunisal is known as a non-selective COX inhibitor or a weak COX-1 selective inhibitor. Referring to Korean Application Publication Patent No. 10-2018-0033426 and 10-2020-0119574, it is described that each of the compound in which diflunisal binds to DO3A containing Gd complex and the compound in which diflunisal binds to DO3A is the selective COX-2 inhibitor and is available as the anti-inflammatory agent. Based on the contents of these two patent documents, it may be identified that the compound in which the diflunisal binds to DO3A is the selective COX-2 inhibitor and thus acts as the anti-inflammatory agent, particularly, as an anti-inflammatory agent for encephalitis diseases. However, as may be identified in the contents of Korean Patent Application Publication No. 10-2020-0119574, the effect thereof as the anti-inflammatory agent is proven, while the effect thereof on thrombus inhibition is not disclosed therein.

### DISCLOSURE

### TECHNICAL PURPOSE

One purpose of the present disclosure is to provide a pharmaceutical composition developed as a conventional anti-inflammatory agent and having a novel medicinal use as an antithrombotic agent.

### TECHNICAL SOLUTION

An antithrombotic agent or thrombolysis agent to achieve the above purpose of the present disclosure includes a compound having a structure represented by a following Chemical Formula 1. The compound having the structure of the following Chemical Formula 1 is a substance known to be conventionally used as an anti-inflammatory agent. In the prior art, cardiovascular side effects were resolved by co-administering the compound with another antithrombotic agent. However, the inventor of the present disclosure has discovered that the compound having the structure of the following Chemical Formula 1 provides a new effect in suppressing the thrombus or achieving the thrombolysis in addition to the anti-inflammatory agent effect. Thus, the compound having the structure of the following Chemical Formula 1 may be be used as a new pharmaceutical use as an antithrombotic agent or thrombolysis agent. 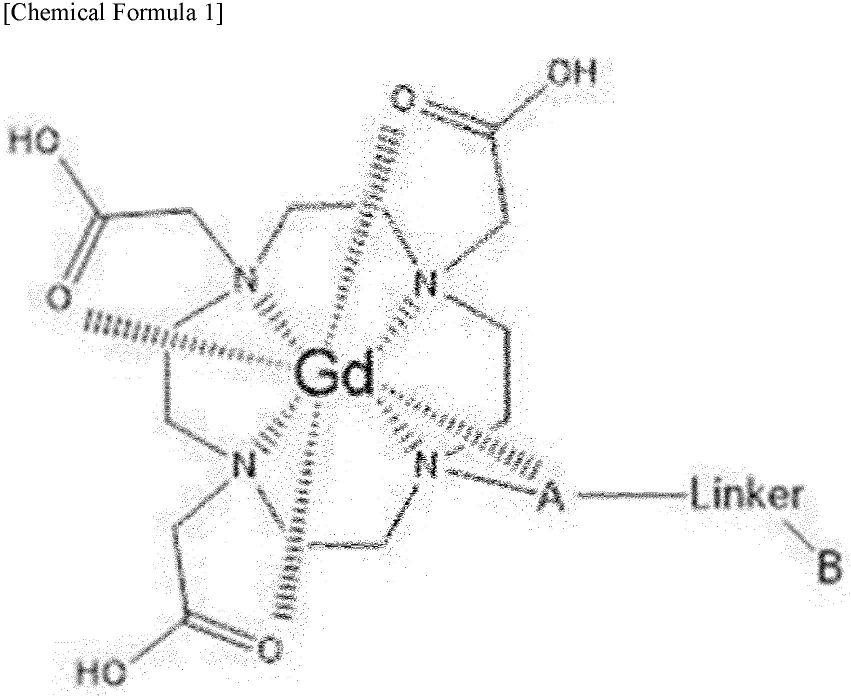 wherein in the Chemical Formula 1,
A represents *-(CH₂)ₙ-A¹-*,
n represents any integer from 0 to 5,
A¹ represents *-NH-*, *-COO-*, *-CO-*, *-NR¹-*, *-CH₂-*, *-CONH-*, or *-O-*,
Linker represents *-L¹-NHCO-L²-*, *-L¹-O-R²-O-L²-*, *-L¹-CH₂-L²-*, *-L¹-NR³-L²-*, or *-L¹-COO-L²-*,
L₁ represents linear or branched (C1-C30) alkyl,
L₂ represents a single bond, or linear or branched (C1-C30) alkyl,
each of R₁ and R₃ independently represents hydrogen or linear or branched (C1-C10) alkyl, R₂ represents linear or branched (C1-C10) alkyl,
B represents 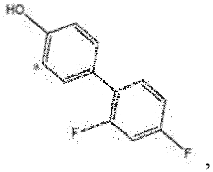 where * represents a binding site.

In one embodiment of the antithrombotic agent or thrombolysis agent, the antithrombotic agent or thrombolysis agent has a structure represented by a following Chemical Formula 2:

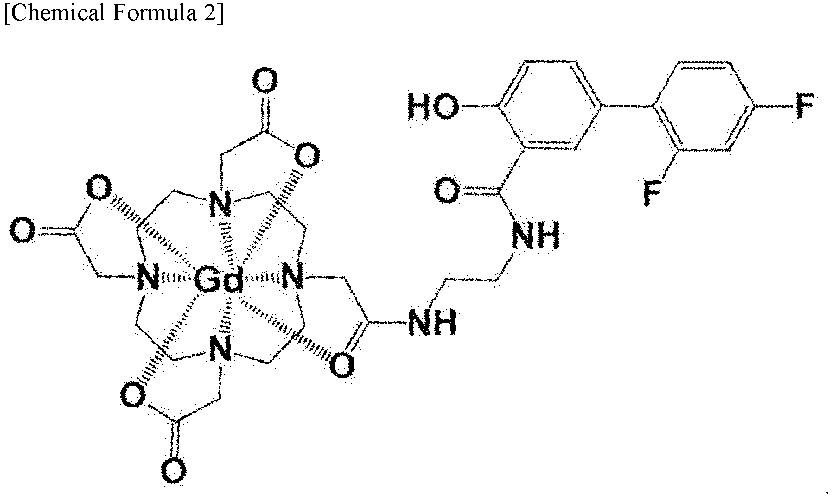

When blood vessels in the body are damaged and bleeding occurs, hemostasis is achieved through the primary and secondary hemostasis processes. Primary hemostasis is hemostasis by platelets in which the platelets attach and aggregate to and on the damaged blood vessel area, and secondary hemostasis is hemostasis by coagulation factors in which firm fibrin is formed in the aggregated platelets via the activation of coagulation factors to completely stop the bleeding. In primary hemostasis, platelets are morphologically transformed from a disc-shape to a spherical shape and are attached to the collagen of blood vessels and thus are aggregated thereon. The attached platelets are activated to release granular substances such as calcium (Ca²⁺) and ADP (adenosine diphosphate). In this regard, the secreted ADP binds to surrounding platelets to activate the platelets, thereby causing the platelets to aggregate with each other. Secondary hemostasis is the formation of firm fibrin via the activation of blood coagulation factors. The activation of blood coagulation factors generates thrombin through the intrinsic pathway and the extrinsic pathway, and thrombin acts on fibrinogen to form fibrin polymers. The fibrin or fibrin polymers bind to the primary hemostatic plug formed by the adhesion and aggregation of the platelets in the primary hemostasis to form a more solid and firm secondary hemostatic plug, thereby achieving complete hemostasis.

The antithrombotic agent or thrombolysis agent of the present disclosure is characterized by inhibiting the platelet aggregation induced by thrombin in the secondary hemostasis. The antithrombotic agent or thrombolysis agent of the present disclosure has the effect of inhibiting the platelet aggregation induced by thrombin in the secondary hemostasis, rather than inhibiting the adhesion of platelets to the damaged area performed in the primary hemostasis.

In one embodiment of the antithrombotic agent or thrombolysis agent, the antithrombotic agent or thrombolysis agent may control integrin αIIBβ3 activation. Integrin αIIBβ3 is a protein involved in adhesion and is a substance that mediates platelet aggregation and interaction with other adjacent tissues. The antithrombotic agent or thrombolysis agent of the present disclosure may suppress the integrin αIIBβ3 activation in a concentration-dependent manner. In the present disclosure, "concentration-dependent" means that the degree of the effect increases as the concentration increases or the degree of the effect decreases as the concentration decreases. That is, as the concentration of the antithrombotic agent or thrombolysis agent of the present disclosure increases, the activity of integrin αIIBβ3 involved in the platelet aggregation may be effectively inhibited.

In one embodiment of the antithrombotic agent or thrombolysis agent, the antithrombotic agent or thrombolysis agent may inhibit the secretion of adenosine triphosphate (ATP). Adenosine triphosphate is a substance released when platelets are activated and may bind to P2Y2 receptors on the surface of vascular endothelial cells. The antithrombotic agent or thrombolysis agent of the present disclosure may affect platelet activity by inhibiting the secretion of ATP.

An anti-inflammatory agent for another purpose of the present disclosure includes a structure represented by a following Chemical Formula 1 and is characterized by being capable of performing thrombus inhibition or thrombolysis. Since the present anti-inflammatory agent performs the thrombus inhibition or thrombolysis, the anti-inflammatory agent of the present disclosure does not require concurrent administration with another drug for the thrombus inhibition. 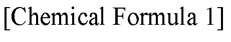 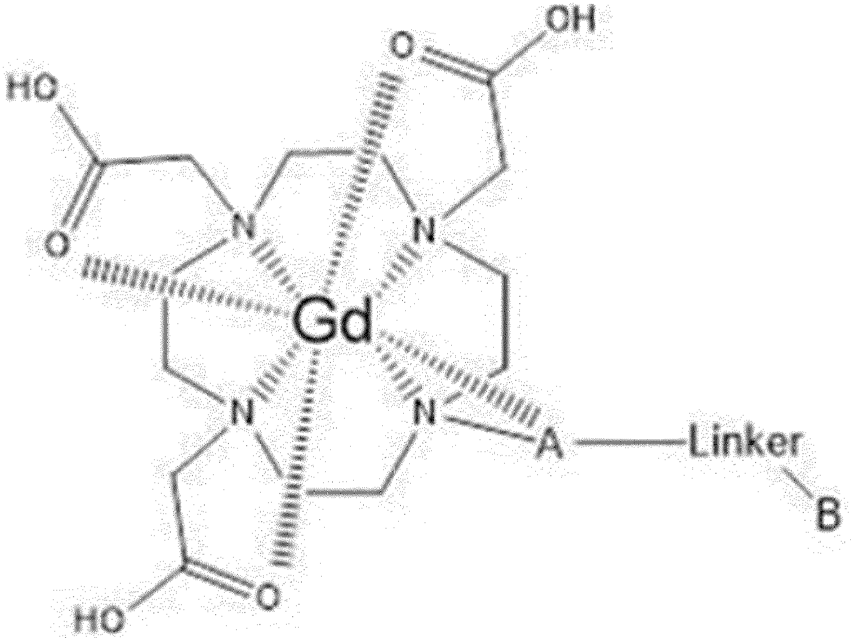 wherein in the Chemical Formula 1,
A represents *-(CH₂)ₙ-A¹*,
n represents any integer from 0 to 5,
A¹ represents *-NH-*, *-COO-*, *-CO-*, *-NR¹*, *-CH₂-*, *-CONH-*, or *-O-*,
Linker represents *-L¹-NHCO-L²-*, *-L¹-O-R²-O-L²-*, *-L¹-CH₂-L²-*, *-L¹-NR³-L²-*, or *-L¹-COO-L²-*,
L₁ represents linear or branched (C1-C30) alkyl,
L₂ represents a single bond, or linear or branched (C1-C30) alkyl,
each of R₁ and R₃ independently represents hydrogen or linear or branched (C1-C10) alkyl, R₂ represents linear or branched (C1-C10) alkyl,
B represents 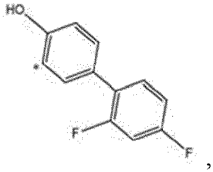 where * represents a binding site.

In one embodiment of the anti-inflammatory agent, the anti-inflammatory agent has a structure represented by a following Chemical Formula 2:

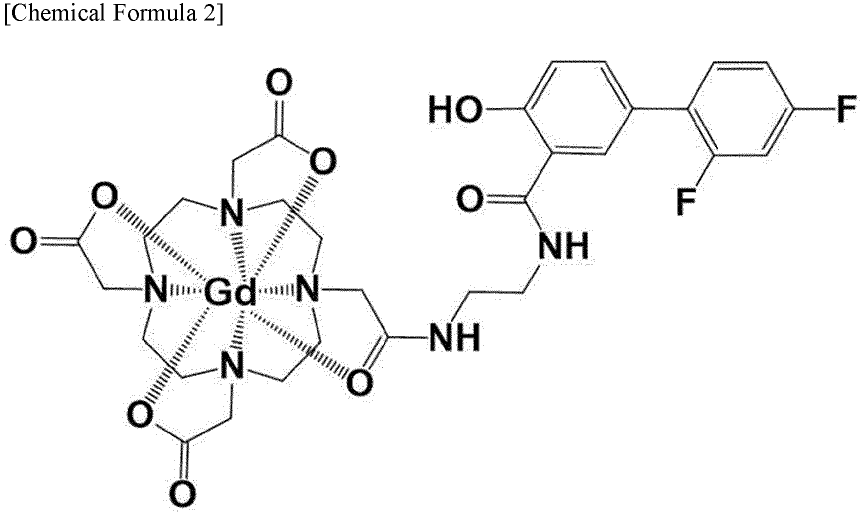

### TECHNICAL EFFECT

According to the present disclosure, a DO3A-based substance containing a gadolinium complex known as an anti-inflammatory agent in the prior art has a novel effect of inhibiting thrombus or performing thrombolysis. In particular, the DO3A-based substance containing the gadolinium complex may effectively inhibit platelet aggregation induced by thrombin. Therefore, the antithrombotic agent or thrombolysis agent of the present disclosure has the advantage of not requiring co-administration with another antithrombotic agent.

### BRIEF DESCRIPTION OF DRAWINGS

FIG. 1 is a diagram for evaluating the platelet aggregation inhibition effect of MBP-11902 of the present disclosure according to Experimental Example 1 of the present disclosure. In the diagram, MBP-11902 is indicated as MBP-02.
FIG. 2 is a diagram for comparing and evaluating the platelet aggregation inhibition efficacy of MBP-11902 of the present disclosure and the platelet aggregation inhibition efficacy of each of NSAID and MBP-11902L as Comparative Examples with each other, according to Experimental Example 2 of the present disclosure. In the diagram, MBP-11902 and MBP-119002L are indicated as MBP-02 and MBP-02L, respectively.
FIG. 3 is a diagram for evaluating the platelet activation efficacy of MBP-11902 of the present disclosure according to Experimental Example 3 of the present disclosure. In the diagram, MBP-11902 is indicated as MBP-02.
FIG. 4 is a diagram for examining the effects of MBP-11902 of the present disclosure on ATP and TXB2 secretion of platelets according to Experimental Example 4 of the present disclosure. In the diagram, MBP-11902 is indicated as MBP-02.
FIG. 5 is a diagram for evaluating the effect of MBP-11902 of the present disclosure on platelet Ca²⁺ behavior (mobilization) according to Experimental Example 5 of the present disclosure. In the diagram, MBP-11902 is indicated as MBP-02.

### DETAILED DESCRIPTIONS

Hereinafter, embodiments of the present disclosure will be described in detail with reference to the accompanying drawings. The present disclosure may be subjected to various changes and may have various forms. Thus, particular embodiments will be illustrated in the drawings and will be described in detail herein. However, this is not intended to limit the present disclosure to a specific disclosed form. It should be understood that the present disclosure includes all modifications, equivalents, and replacements included in the spirit and technical scope of the present disclosure. While describing the drawings, similar reference numerals are used for similar components.

The terminology used herein is directed to the purpose of describing particular embodiments only and is not intended to be limiting of the present disclosure. As used herein, the singular constitutes "a" and "an" are intended to include the plural constitutes as well, unless the context clearly indicates otherwise. It will be further understood that the terms "comprise", "including", "include", and "including" when used in this specification, specify the presence of the stated features, integers, operations, elements, and/or components, but do not preclude the presence or addition of one or more other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, the DO3A-based antithrombotic agent or thrombolysis agent containing the gadolinium complex of the present disclosure will be described in more detail through specific Present Examples and Comparative Examples. However, the Examples of the present disclosure are only some embodiments of the present disclosure, and the scope of the present disclosure is not limited to the following Examples.

### Present Example 1 <MBP-11902>

The compound used in Present Example 1 of the present disclosure (hereinafter, MBP-11902) is provided from Etnova Therapeutics Co., Ltd. MBP-11902 of Present Example 1 of the present disclosure is indicated as MBP-02 in all diagrams.

### Comparative Example 1 <NSAID>

The compound used as Comparative Example 1 of the present disclosure (hereinafter, NSAID) is Diflunisal provided from Etnova Therapeutics Co., Ltd.

### Comparative Example 2 <MBP-11902L>

The compound used as Comparative Example 2 of the present disclosure (hereinafter, MBP-11902L) is a ligand compound in which gadolinium has been removed from the compound of Present Example 1 of the present disclosure, and is provided from Etnova Therapeutics Co., Ltd. MBP-11902L of Comparative Example 2 of the present disclosure is indicated as MBP-02L in all diagrams.

### Experimental Example 1

### ① Evaluation of platelet aggregation inhibition efficacy of MBP-11902

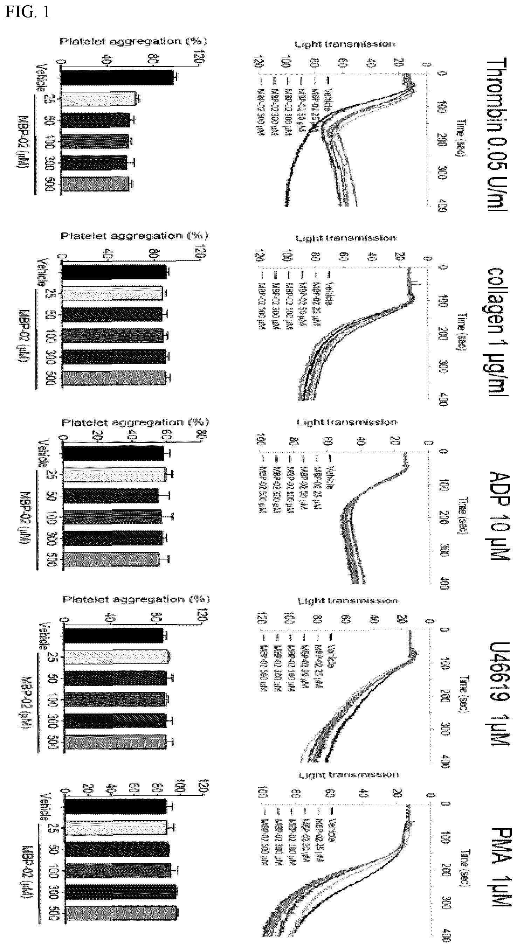

In order to evaluate the platelet aggregation activity of MBP-11902 according to Present Example 1 of the present disclosure, the platelet aggregation was activated in rat plasma with platelet aggregation factors and agonists, thrombin, collagen, ADP (adenosine diphosphate), U46619 (thromboxane A2 agonist), and PMA (phorbol-12-myristate-13-acetate), and then, the rat plasma was pretreated with vehicle (distilled water) and various doses (100, 300, and 500 µM) of MBP-11902, and then a 4-channel platelet lumi-aggregometer (Chronolog Corp, Havertown, PA) was used to measure the value indicating the platelet aggregation level, and the results are shown in FIG. 1.

FIG. 1 is a diagram for evaluating the platelet aggregation inhibition efficacy of MBP-11902 of the present disclosure according to Experimental Example 1 of the present disclosure.

Referring to FIG. 1, it was observed that compared to the control (vehicle), MBP-11902 of the present disclosure effectively inhibited the platelet aggregation induced by thrombin (0.05 U/mL). In this regard, when observing the platelet aggregation effect based on the concentration of MBP-11902 (25, 50, 100, 300, and 500 µM), it was observed that there was no significant difference in the inhibition effect based on the concentration. Further, it was not observed that MBP-11902 of the present disclosure inhibited the platelet aggregation induced by collagen (1 µg/mL), ADP (10 µM), U46619 (1 µM), and PMA (1 µM).

In summary, it was identified that MBP-11902 of the present disclosure inhibited the platelet aggregation induced only by thrombin, but did not inhibit the platelet aggregation in a concentration-dependent manner. This suggests that MBP-11902 exhibits platelet aggregation inhibition efficacy through the secondary signaling pathway (signaling pathway) in platelet aggregation mediated by thrombin, but has limitations in inhibiting platelet aggregation through platelet morphological transformation and a primary signaling pathway.

### ② Comparative evaluation of platelet aggregation inhibition efficacies of MBP-11902, NSAID, and MBP-11902L

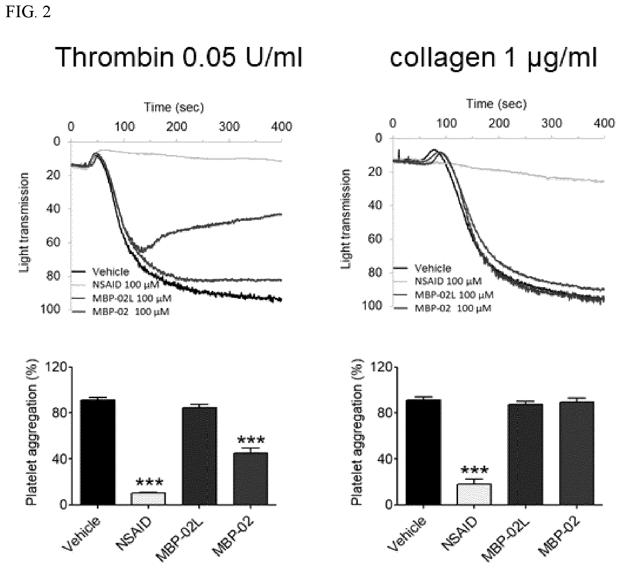

In order to reevaluate the inhibition efficacy of the platelet aggregation mediated only by thrombin of MBP-11902 regarding the platelet aggregation inhibition efficacy of MBP-11902 in Experimental Example 1, the platelet aggregation efficacy of each of the original NSAID, the intermediate MBP-11902L, and the product MBP-11902 (each 100 µM) was evaluated. The evaluation was performed using thrombin and collagen, and the 4-channel platelet lumi-aggregometer (Chronolog Corp, Havertown, PA) was used to measure the value indicating the platelet aggregation level, and the results are shown in FIG. 2.

FIG. 2 is a diagram for comparing and evaluating the platelet aggregation inhibition efficacy of MBP-11902 of the present disclosure and the platelet aggregation inhibition efficacy of each of Comparative Examples NSAID and MBP-11902L with each other according to Experimental Example 2 of the present disclosure.

Referring to FIG. 2, NSAID (100 µM) was observed to significantly inhibit platelet aggregation induced by thrombin (0.05 U/ml) and collagen (1 µg/mL), compared to the control (vehicle). On the other hand, the intermediate MBP-11902L (100 µM) was observed to have no effect of inhibiting the platelet aggregation induced by each of thrombin (0.05 U/ml) and collagen (1 µg/mL), compared to the control (vehicle). Further, MBP-11902 of the present disclosure was identified to inhibit platelet aggregation induced only by thrombin.

In summary, the original NSAID may significantly inhibit the platelet activity, the intermediate MBP-11902L has no effect on platelet aggregation inhibition, and the MBP-11902 of the present disclosure inhibits the platelet aggregation induced only by thrombin only in the second signaling pathway. Therefore, the original NSAID is excellent in inhibiting the platelet aggregation. However, using the NSAID, the side effect of bleeding may occur. However, MBP-11902 of the present disclosure exhibits the platelet aggregation inhibition efficacy only through the secondary signaling pathway mediated by thrombin in the platelet aggregation. Thus, it is thought that compared to the original NSAID and the intermediate MBP-11902L, MBP-11902 of the present disclosure may inhibit the platelet aggregation without side effects of the bleeding in the platelet aggregation mediated by thrombin.

### ③ Evaluation of platelet activation efficacy of MBP-11902

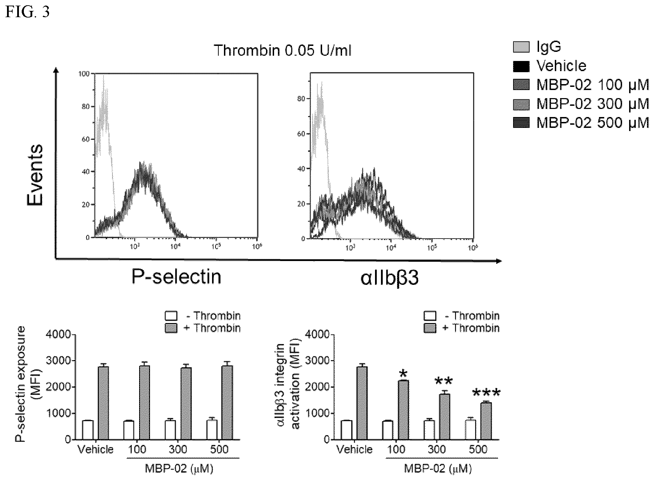

In the platelet aggregation inhibition experiment of Experimental Example 1 as set forth above, it was identified that the MBP-11902 selectively exhibited the inhibition effect of the platelet aggregation in the signaling pathway mediated by thrombin. Therefore, in this platelet activation experiment, an activation efficacy inhibition experiment was conducted using only thrombin. The efficacy of MBP-11902 on the regulation mechanism of activation of α-granule, P-selectin, and integrin αIIBβ3 used as the platelet activation biomarker in a major process that induces a positive feedback cycle in the platelet activation was evaluated using flow cytometry analysis (FCM), and the results are shown in FIG. 3.

FIG. 3 is a diagram for evaluating the platelet activation efficacy of MBP-11902 of the present disclosure according to Experimental Example 3 of the present disclosure.

Referring to FIG. 3, compared to the control (vehicle), MBP-11902 of the present disclosure was observed to significantly inhibit the activation of rat platelet integrin αIIBβ3 in a concentration-dependent manner (100, 300, and 500 µM), but was observed to have no inhibitory effect on the activities of α-granule and P-selectin.

This result suggests that MBP-11902 of the present disclosure selectively inhibits platelet-platelet aggregation activity through the activation of integrin αIIbβ3 present on the platelet cell membrane without affecting platelet granule secretion.

### ④ Evaluation of platelet activity inhibition by MBP-11902 via ATP and TXB2 secretion measurements

When the platelet aggregation occurs, ATP (Adenosine triphosphate) and TXB2 (Thromboxane B2) are released, which activate the intrinsic receptors of the outer wall of platelet cells through autocrine and paracrine secretion, thereby promoting platelet aggregation. Therefore, ATP and TXB2 secretion and activation are known as the main mechanisms of thrombus aggregation reaction in platelet aggregation reaction.

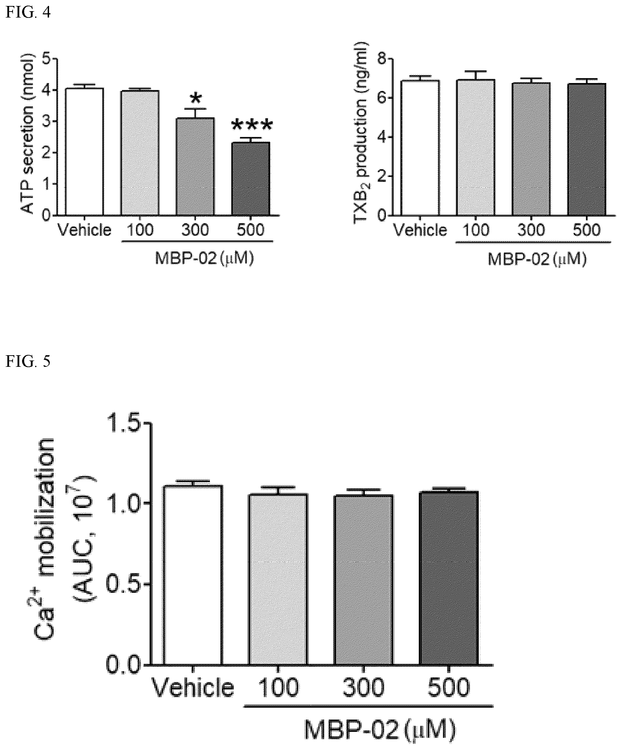

In accordance with the present disclosure, in order to identify the ATP and TXB2 secretion activity of MBP-11902, platelet aggregation activity was increased with thrombin, followed by treatment with MBP-11902 at various concentrations (100, 300, and 500 µM). Then, the ATP and TXB2 production concentrations were measured using luciferin/luciferase reagent (Chronolog) and a TXB2 production assay kit, and the results are shown in FIG. 4.

FIG. 4 is a diagram to determine the effect of MBP-11902 of the present disclosure on ATP and TXB2 secretion of platelets according to Experimental Example 4 of the present disclosure.

Referring to FIG. 4, compared to the control (vehicle), it was identified that the rat platelet ATP secretion only from the platelets treated with MBP-11902 was significantly inhibited in a concentration-dependent manner. On the other hand, there was no significant difference in TXB2 generation. These results indicate that MBP-11902 of the present disclosure affects the platelet activity through regulation of ATP secretion in platelet activity, but does not affect TXB2 generation.

### ⑤ Evaluation of effect of MBP-11902 on platelet Ca²⁺ behavior (mobilization)

Platelet agonists stimulate other receptors of platelets to release intracellular Ca²⁺ or to accept Ca²⁺ from the outside out of the platelet, thereby causing Ca²⁺ behavior (mobilization) in the platelet cytoplasm. The basic mechanism of this Ca²⁺ behavior is important for the platelet activation, including platelet α-granule, P-selectin, and integrin αIIBβ3 activation regulation mechanisms. Therefore, it was investigated whether MBP-11902 of the present disclosure plays an important role in Ca²⁺ behavior during platelet activation.

Rat platelets (1 × 10⁸/mL) were treated with the control (vehicle) or various doses (100, 300, and 500 µM) of MBP-11902 at 37°C for 10 mins, and then incubated with the FLIPR Calcium 5 Assay kit at 37°C for 30 mins in a light-shielded location, and then, the platelets were activated with thrombin. Cytoplasmic Ca²⁺ concentration was measured using a spectrofluorometer (Spectramax I3, Molecular Devices) with an input wavelength of 485 nm and an emission wavelength of 525 nm, and the results are shown in FIG. 5.

FIG. 5 is a diagram for evaluating the effect of MBP-11902 of the present disclosure on platelet Ca²⁺ behavior (mobilization) according to Experimental Example 5 of the present disclosure.

Referring to FIG. 5, it was observed that the Ca²⁺ behavior of platelets was not controlled by MBP-11902. This result suggests that platelet activation according to thrombin-induced Ca²⁺ concentration change is not regulated by MBP-11902.

The results of Experimental Examples 1 to 5 of the present disclosure are summarized as follows.

MBP-11902 of the present disclosure does not affect the thrombin-mediated platelet activation, but significantly inhibits platelet-platelet aggregation, and thus plays an important role in secondary platelet-platelet aggregation inhibition rather than primary platelet-mediated thrombus inhibition. It is identified that MBP-11902 of the present disclosure does not affect the hemostasis but performs the thrombolysis without not causing the side effects of the existing NSAID. Therefore, MBP-11902 of the present disclosure may be applied as a functional raw material or therapeutic agent for improving the blood flow based on its effect on thrombolysis.

### <Materials and Analysis Method for Experimental Examples 1 to 5>

### ① Materials

Human thrombin, thromboxan A2 analogue (U46619), PMA, PGE1, dimethyl sulfoxide (DMSO), ADP, fibrinogen, and all reagents were purchased from Sigma (St. Louis, MO). D-Phe-Pro-Arg-chloromethyl ketone (PPACK) was purchased from EMD Millipore (Billerica, MA). Phycoerythrin (PE)-conjugated isotype control IgGs, PE anti-mouse CD62P (P-selectin) antibody, and PE anti-mouse αIIbβ3 (JON/A) antibody were purchased from Emfret Analytics (Eibelstadt, Germany). The FLIPR Calcium Assay Kit as a Ca²⁺ indicator was purchased from Molecular Devices (Sunnyvale, CA, USA) and used in this experiment.

### ② Platelet isolation method

Platelets from male C57BL/6 (6-8 weeks) were collected from the abdominal vein using a syringe pretreated with citrate-dextrose solution (ACD, Sigma). To obtain platelet-rich plasma (PRP), the plasma was centrifuged at 300×g for 20 mins at room temperature, and then, plasma separated from red blood cells was added thereto at 0.5 µM PGE1, followed by re-centrifugation at 700×g for 4 mins. Then, the precipitated platelets were washed with HEPES-Tyrode buffer (5 mM HEPES/NaOH, pH 7.3, 5 mM glucose, 136 mM NaCl, 12 mM NaHCO3, 2.7 mM KCl) containing 10% ACD, followed by re-centrifugation at 700×g for 5 mins. Then, a concentration of the precipitated platelets was adjusted to a concentration of 3 × 10⁸ cells/mL in HEPES-Tyrode buffer and the resulting platelets were used in this experiment.

### ③ Platelet aggregation analysis method

Rat platelets were placed in HEPES-Tyrode buffer at a concentration of 3 x 10⁸ cells/mL and pretreated with the vehicle (distilled water) and various doses (100, 300, and 500 µM) of MBP-11902 at 37°C for 15 minutes, and then activated with collagen, thrombin, ADP, U46619 (endoperoxide/thromboxane analogs), and PMA (PKC activator), which are known as agonists in the platelet aggregation system. The platelet aggregation was induced at 1,000 rpm in a platelet aggregation system (Chronolog Corp, Havertown, PA) at 37°C and then, the platelet aggregation level was measured.

### ④ Measurement of ATP secretion

Rat platelets were pretreated with the vehicle (distilled water) and various doses (100, 300, and 500 µM) of MBP-11902 at 37°C for 10 mins, and then activated with thrombin as one of the platelet aggregation agonists at 37°C. The platelet activation reaction was terminated by centrifugation, and the supernatant was collected to measure the amount of adenosine triphosphate (ATP) secretion. ATP secretion was measured using an ATP assay kit (Biomedical Research Service Center, Buffalo, NY) and with a GloMax^{®} Explorer Multimode Microplate Reader (Promega).

### ⑤ Measurement of TXB2 production

Rat platelets were pretreated with the vehicle (distilled water) and various doses (100, 300, and 500 µM) of MBP-11902 at 37°C for 10 mins, and then activated with thrombin as one of the platelet aggregation agonists at 37°C. Platelet activation was stopped after 5 mins by adding 2 mM EGTA containing 0.1 M KCl and 5 mM indomethacin thereto. The mixture was centrifuged at 6,000×g for 3 mins, and the supernatant was collected to measure the thromboxane (TXB2) production level using a TXB2 production kit (Enzo Life Sciences, Farmingdale, NY).

### ⑥ Flow cytometry analysis (FCM)

The activation of P-selectin and integrin αIIBβ3 known as biomarkers of the platelet activity was measured using a flow cytometer (Gallios, Beckman Coulter). Rat platelets were pretreated with the vehicle (distilled water) and various doses (100, 300, and 500 µM) of MBP-11902 for 15 minutes at 37°C. For the platelet activity, the rat platelets were treated with thrombin for 5 minutes at 37°C, and then the activity was measured using P-selectin and integrin αIIBβ3.

### ⑦ Ca²⁺ Behavior Analysis

Rat platelets (1 × 10⁸ cells/mL) were suspended in HEPES-Tyrod buffer free of CaCl₂ and pretreated with the vehicle (distilled water) and various doses (100, 300, and 500 µM) of MBP-11902 for 10 mins at 37°C. The rat platelets were stained with Ca²⁺ dye (FLIPR Ca²⁺ 5 analysis kit) for 30 mins in the dark environment and activated with 0.05 U/mL thrombin. A Ca²⁺ level in the platelets was measured using a spectrofluorometer (Spectramax I3, Molecular Devices) at a wavelength of 505 nm.

### ⑧ Statistical processing

The statistical analysis of the data was performed using GraphPad Prism 5. The statistical significance was analyzed with ANOVA of multi-groups and Dunnett's test for comparisons of multiple groups, and Student's t-test for comparisons of two groups. A P value lower than 0.05 was considered significant.

Although the present disclosure has been described above with reference to preferred embodiments of the present disclosure, those skilled in the art will understand that the present disclosure may be variously modified and changed within the scope that does not depart from the spirit and scope of the present disclosure described in the following patent claims.

## Claims

1. An antithrombotic agent or thrombolysis agent having a structure represented by a following Chemical Formula 1: 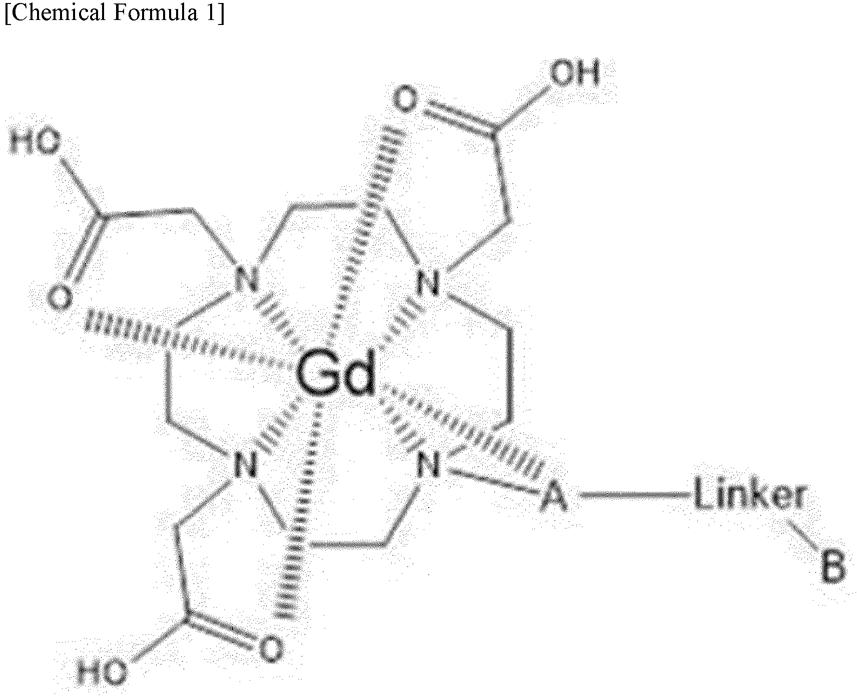 wherein in the Chemical Formula 1,
A represents *-(CH₂)ₙ-A¹*,
n represents any integer from 0 to 5,
A¹ represents *-NH-*, *-COO-*, *-CO-*, *-NR¹*, *-CH₂-*, *-CONH-*, or *-O-*,
Linker represents *-L¹-NHCO-L²-*, *-L¹-O-R²-O-L²-*, *-L¹-CH₂-L²-*, *-L¹-NR³-L²-*, or *-L¹-COO-L²-*,
L₁ represents linear or branched (C1-C30) alkyl,
L₂ represents a single bond, or linear or branched (C1-C30) alkyl,
each of R₁ and R₃ independently represents hydrogen or linear or branched (C1-C10) alkyl,
R₂ represents linear or branched (C1-C10) alkyl,
B represents 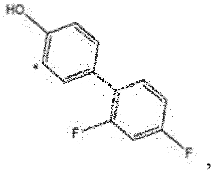 where * represents a binding site.

2. The antithrombotic agent or thrombolysis agent of claim 1, wherein the antithrombotic agent or thrombolysis agent has a structure represented by a following Chemical Formula 2:

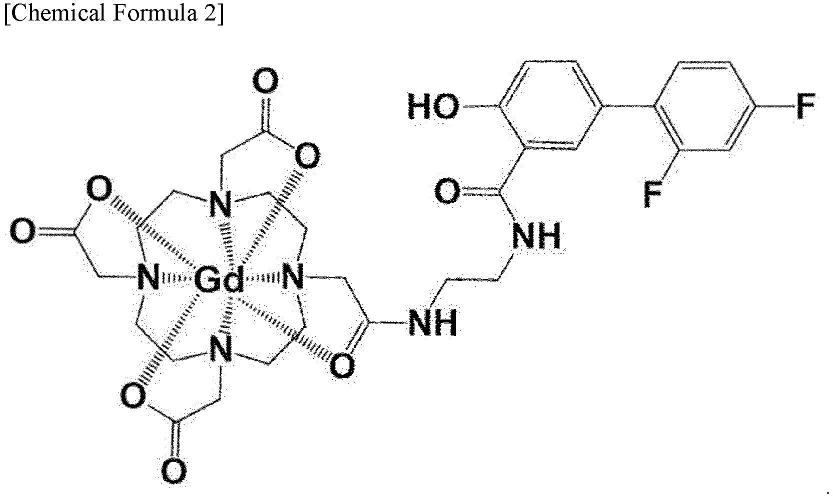

3. The antithrombotic agent or thrombolysis agent of claim 1, wherein the antithrombotic agent or thrombolysis agent inhibits platelet aggregation induced by thrombin.

4. The antithrombotic agent or thrombolysis agent of claim 3, wherein the antithrombotic agent or thrombolysis agent inhibits integrin αIIBβ3 activation in a concentration-dependent manner.

5. The antithrombotic agent or thrombolysis agent of claim 3, wherein the antithrombotic agent or thrombolysis agent inhibits secretion of ATP (Adenosine triphosphate).

6. An anti-inflammatory agent having a structure represented by a following Chemical Formula 1 and capable of inhibiting thrombus or capable of thrombolysis: 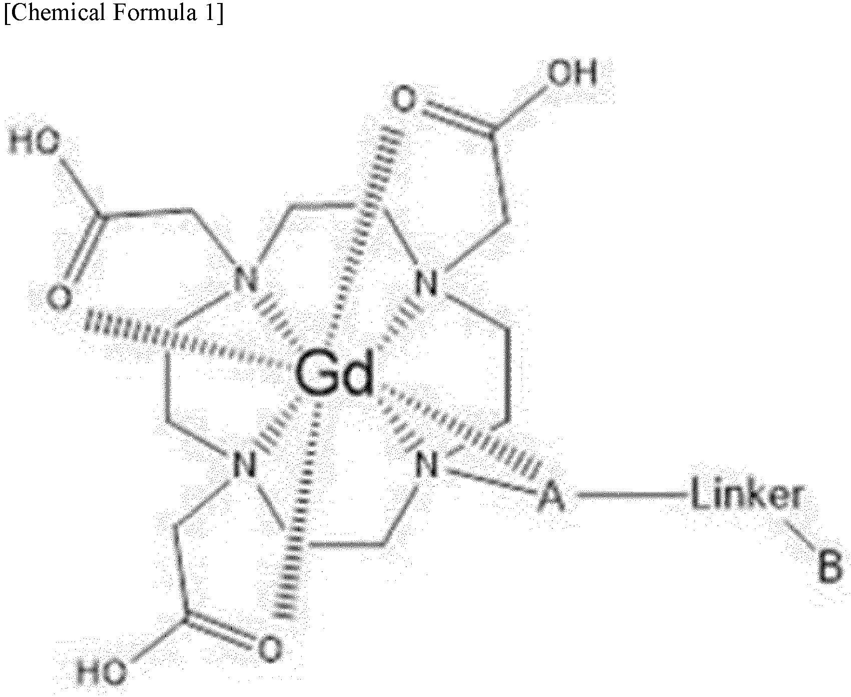 wherein in the Chemical Formula 1,
A represents *-(CH₂)ₙ-A¹*,
n represents any integer from 0 to 5,
A¹ represents *-NH-*, *-COO-*, *-CO-*, *-NR¹*, *-CH₂-*, *-CONH-*, or *-O-*,
Linker represents *-L¹-NHCO-L²-*, *-L¹-O-R²-O-L²-*, *-L¹-CH₂-L²-*, *-L¹-NR³-L²-*, or *-L¹-COO-L²-*,
L₁ represents linear or branched (C1-C30) alkyl,
L₂ represents a single bond, or linear or branched (C1-C30) alkyl,
each of R₁ and R₃ independently represents hydrogen or linear or branched (C1-C10) alkyl,
R₂ represents linear or branched (C1-C10) alkyl,
B represents 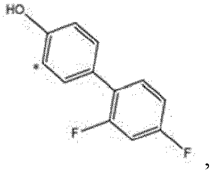 where * represents a binding site.

7. The anti-inflammatory agent of claim 6, wherein the anti-inflammatory agent has a structure represented by a following Chemical Formula 2:
